# EUROPEAN PATENT SPECIFICATION

(11) **EP 1 181 351 B1**
(45) Date of publication and mention of the grant of the patent: **12.11.2008**
(21) Application number: 00922208.4
(22) Date of filing: 14.04.2000
(51) Int. Cl.: C12N 5/00, C12N 15/00, C12P 21/00, A01K 67/027

(54) **METHOD OF PURIFYING HETEROLOGOUS PROTEINS**
VERFAHREN ZUR AUFREINIGUNG HETEROLOGER PROTEINE
PURIFICATION DE PROTEINES HETEROLOGUES

(30) Priority: 14.04.1999 US 129299 P
(43) Date of publication of application: 27.02.2002
(73) Proprietor: GTC Biotherapeutics, Inc., Framingham, MA 01702 (US)
(72) Inventor: MEADE, Harry, Newton, MA 02158 (US)
(74) Representative: Ruffles, Graham Keith
(86) International application number: PCT/US2000/010095
(87) International publication number: WO 2000/061725

(56) References cited:
- EP-A- 0 881 288
- US-A- 4 873 316
- US-A- 5 322 775
- US-A- 5 523 226
- US-A- 5 589 604
- US-A- 5 633 076

## Description

This application claims the benefit of a previously filed Provisional Application No. 60/129,299, filed April 14, 1999.

### Background of the Invention

Milk from domestic animals has been used as a source of proteins and other products for the food and pharmaceutical industries for many years. Among ruminants and laboratory animals, milk contains an average of 30 to 140 grams of protein per liter, or about 4-17% by weight, depending on the species. The bulk of these proteins are caseins, which are complexed with calcium and phosphate in supramolecular structures known as micelles. The other major class of milk proteins is whey proteins, predominantly comprised of beta-lactoglobulin and alpha-lactalbumin, but also including lactoferrin, immunoglobulins, and serum albumin.

More recently, methods have become known in the art for expressing exogenous proteins at commercially feasible levels in the milk of transgenic animals. Exogenous protein expression levels generally range from less than 1 to 10 or more grams per liter, depending on the protein and the species. Commercial production of a wide range of proteins in the milk of transgenic livestock is now under development (A. J. Clark, et al., Trends in Biotechnology, 5:20-24, 1987, A.J. Clark, Journal of Cellular Biochemistry, 49:121-127,1992; W. Bawden et al., Biotechnology and Genetic Engineering Reviews, 12:89-137, 1994; N. S. Rudolph, Genetic Engineering News, 15:8-9, 1995). Exogenous peptides, and in particular human peptides, may be produced in milk at relatively high concentrations and in large volumes, providing continuous high-level output of normally processed peptides.

US patent 5,633,076 discloses the production of human serum albumin (hSA) by a transgenic mouse, with the exogenous gene being inserted into the mouse genome by homologous recombination. This results in the deletion of the endogenous form of the gene.

EP 08801288 describes the expression of a tagged exogenous protein in a mouse system, achieved by knockout of the endogenous protein. The tag is used purely to identify transgenic animals during their creation; there is no suggestion that the tagging my be used in order to isolate one version of the protein from a native protein produced by the animal.

Although it is possible to produce high levels of exogenous peptides in the mammary gland, the milk also contains significant levels of the animal's own serum proteins. It has been shown that serum proteins are present in milk at 2% of their serum levels. Thus, methods are needed which allow for separation of recombinant proteins secreted into the milk of a transgenic animal from an animal's own serum proteins.

### Summary of the Invention

The present invention is based, in part, on the discovery of a separation method that can distinguish between two almost identical versions of a protein, e.g., a human protein and the protein of a non-human host cell, e.g., of a transgenic non-human host, e.g., a dairy animal host. It was found that the host protein can be separated from a heterologous protein produced in the milk of the non-human animal by altering the host endogenous protein.

It is often necessary to purify a heterologous protein free of the animal's version of the protein. Generally, it is estimated that the host serum proteins are found in milk at 2% of their serum level. For many other proteins, the levels are in the microgram range. For example, goat antithrombin III (ATIII) levels in milk are less than 3 µg/ml. It is, however, necessary to remove the host animal's version of the protein to levels far below that level. In addition, many proteins have a high level of homology across species barriers which can impede methods of separating a host animal's version of a protein and a heterologous protein expressed by that animal. For example, human serum albumin (HSA) is very similar to bovine serum albumin (BSA). It was found that by altering the host animal's version of the protein, a heterologous protein, e.g., a heterologous protein secreted into the milk of the animal, can be distinguished from and separated from the animal's endogenous protein.

Accordingly, in one aspect, the invention features a method of providing an exogenous polypeptide expressed by a non-human transgenic mammal in accordance with claim 1.

In a preferred embodiment, the method can further include separating the altered protein from the sample, e.g., a tissue or a fluid (e.g., milk, blood, urine).

In a preferred embodiment, the non-human transgenic mammal is homozygous for the gene which encodes the altered protein. In another preferred embodiment, the non-human transgenic animal is heterozygous for the gene which encodes the altered protein, e.g., the altered protein is a dominantly expressed protein.

In a preferred embodiment, the protein naturally expressed in the non-human transgenic mammal is altered by replacing the gene encoding the protein with a DNA sequence encoding the heterologous polypeptide, e.g., a cDNA sequence or a genomic sequence encoding the heterologous polypeptide. In a preferred embodiment, a cDNA encoding the heterologous polypeptide is inserted prior to the initiation codon of the gene encoding the naturally expressed protein with or without removing the sequence coding for the naturally expressed protein from the genome. If the sequence coding for the naturally expressed protein is not removed, the signal sequence of the protein can be removed and/or a termination sequence can be inserted at the 3' end of the cDNA sequence encoding the heterologous polypeptide. Thus, replacement means "replacement of expression" and is not linked to an "exchange" of one gene for another in the genome.

In another preferred embodiment, the gene encoding the naturally expressed protein is replaced with a DNA sequence, e.g., the genomic sequence, encoding the heterologous polypeptide, e.g., the sequence encoding the naturally expressed protein is removed and replaced with, e.g., the genomic sequence, which encodes the heterologous polypeptide. In a preferred embodiment, the sequence encoding the heterologous protein is inserted: after the 5' untranslated region (UTR) of the naturally expressed protein; prior to the 3' UTR of the naturally expressed protein; in between the 5' UTR and the 3' UTR of the naturally expressed protein.

In a preferred embodiment, the protein naturally expressed in the non-human transgenic mammal is altered by changing the sequence of the gene, e.g., by adding, deleting, or substituting a nucleotide, such that the protein is changed. The protein can be changed by, for example, deleting at least one amino acid, adding at least one amino acid, or the substitution of at least one amino acid. A nucleotide can be added at the C-terminus, the N-terminus, or at internal points within the gene, e.g., to achieve the change in the protein. In a preferred embodiment, the protein naturally expressed in the non-human transgenic mammal is changed by adding at least one amino acid to the protein, e.g., an added amino acid or amino acids that can bind to a preselected ligand, e.g., a 6X HIS ligand, a cellulose binding domain (CBD) ligand, a maltose binding protein (MBP) ligand. In a referred embodiment, the protein naturally expressed in the transgenic mammal is changed by addition of an affinity tag to the gene encoding the naturally expressed protein. In a preferred embodiment, more than 1-100, 5-50, 10-30 amino acid residues can be added to the protein. The residues allow for a physical difference between the altered protein and the transgenic product and are useful, e.g., in purification.

In a preferred embodiment, the protein naturally expressed in the non-human transgenic mammal is altered by changing the gene encoding the protein such that the stop codon of the protein is altered, e.g., the stop codon is altered by substitution of at least one nucleotide in the stop codon. In another preferred embodiment, the stop codon is altered by removal of at least one nucleotide or addition of at least one nucleotide in the sequence encoding the stop codon.

In a preferred embodiment, the protein naturally expressed in the non-human transgenic mammal is altered by changing an amino acid sequence of the protein such that the stop codon is altered, e.g., the stop codon is altered by addition of at least one amino acid.

In a preferred embodiment, the transgenic mammal is selected from the group consisting of a goat, a bovine, a sheep, a pig, a horse, a mouse, and a rat. Preferably, the transgenic mammal is a bovine or a goat.

In a preferred embodiment, the heterologous polypeptide is a human polypeptide. In a preferred embodiment, the heterologous polypeptide is: a serum protein, a milk protein, a glycosylated or a nonglycosylated protein. A heterologous polypeptide includes, but is not limited to, any of: alpha proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and alpha-antitrypsin.

In a preferred embodiment, the heterologous sequence is under the control of a promoter, e.g., a tissue-specific promoter (e.g., a milk-specific promoter, a blood-specific promoter, or a mine-specific promoter). A milk-specific promoter can be any of: a casein promoter, a beta lactoglobulin promoter, a whey acid protein promoter, and a lactalbumin promoter.

Also described is a method of providing a heterologous polypeptide expressed by a non-human transgenic mammal. The method includes: providing a non-human transgenic mammal which includes a transgenic sequence encoding the heterologous polypeptide and which expresses an altered form of the protein naturally expressed by the transgenic mammal, wherein gene encoding the naturally expressed protein is replaced with the sequence encoding the heterologous polypeptide; and obtaining a sample from the transgenic mammal, wherein the sample includes the heterologous polypeptide and the altered protein, to thereby provide a heterologous polypeptide.

Also described is a method of providing a heterologous polypeptide expressed by a non-human transgenic mammal. The method includes: providing a non-human transgenic mammal which includes a transgenic sequence encoding the heterologous polypeptide and which expresses an altered form of the protein naturally expressed by the transgenic mammal, wherein gene encoding the naturally expressed protein is modified, e.g., by addition, deletion, or substitution of at least one nucleotide, such that the amino acid sequence is altered, e.g., by the addition, deletion, or substitution of at least one amino acid; and obtaining a sample from the transgenic mammal, wherein the sample includes the heterologous polypeptide and the altered protein; to thereby provide the heterologous polypeptide.

Also described is a method of providing a heterologous polypeptide expressed by a non-human transgenic mammal. The method includes: providing a non-human transgenic mammal which includes a transgenic sequence encoding the heterologous polypeptide and which expresses an altered form of the protein naturally expressed by the transgenic mammal, wherein the naturally expressed protein is altered such that the stop codon in the protein is altered, e.g., by addition, deletion or substitution of an amino acid to the stop codon; and obtaining a sample from the transgenic mammal, wherein the sample includes the heterologous polypeptide and the altered protein, to thereby provide the heterologous polypeptide.

Also described is a method of providing human serum albumin expressed by a non-human transgenic mammal. The method includes: providing a non-human transgenic mammal which includes a transgene encoding human serum albumin and which expresses an altered form of the serum albumin naturally expressed by the transgenic mammal; and, obtaining a sample from the transgenic mammal, wherein the sample includes human serum albumin and the altered serum albumin, to thereby provide human serum albumin.

Also described is a method of providing human serum albumin expressed by a transgenic bovine. The method includes: providing a transgenic bovine which includes a transgene encoding human serum albumin and which expresses an altered form of bovine serum albumin; obtaining a sample from the transgenic bovine, wherein the sample includes human serum albumin and the altered bovine serum albumin, to thereby provide human serum albumin.

Also described is a method of providing a heterologous polypeptide expressed by a host cell. The method includes:
providing a cell which includes a nucleotide sequence encoding the heterologous polypeptide and which expresses an altered form of the protein naturally expressed by the cell; and, obtaining a sample from the cell, wherein the sample comprises the heterologous polypeptide and the altered protein, to thereby provide the heterologous polypeptide.

In a preferred embodiment, the method can further include separating the altered protein from the sample, e.g., separating the altered protein from the culture media.

In a preferred embodiment, the cell is homozygous for the gene which encodes the altered protein. In another preferred embodiment, the cell is heterozygous for the gene which encodes the altered protein, e.g., the altered protein is a dominantly expressed protein.

In a preferred embodiment, the protein naturally expressed in the cell is altered by replacing the gene encoding the protein with a DNA sequence encoding the heterologous polypeptide, e.g., a cDNA sequence or a genomic sequence encoding the heterologous polypeptide. In a preferred embodiment, a cDNA encoding the heterologous polypeptide is inserted prior to the initiation codon of the gene encoding the naturally expressed protein with or without removing the sequence coding for the naturally expressed protein from the genome. If the sequence coding for the naturally expressed protein is not removed, the signal sequence of the protein can be removed and/or a termination sequence can be inserted at the 3' end of the cDNA sequence encoding the heterologous polypeptide. Thus, replacement means "replacement of expression" and is not linked to an "exchange" of one gene for another in the genome.

In another preferred embodiment, the gene encoding the naturally expressed protein is replaced with a DNA sequence, e.g., the genomic sequence, encoding the heterologous polypeptide, e.g., the sequence encoding the naturally expressed protein is removed and replaced with, e.g., the genomic sequence, which encodes the heterologous polypeptide. In a preferred embodiment, the sequence encoding the heterologous protein is inserted: after the 5' untranslated region (UTR) of the naturally expressed protein; prior to the 3' UTR of the naturally expressed protein; in between the 5' UTR and the 3' UTR of the naturally expressed protein.

In a preferred embodiment, the protein naturally expressed in the cell is altered by changing the sequence of the gene, e.g., by adding, deleting, or substituting a nucleotide, such that the protein is changed. The protein can be changed by, for example, deleting at least one amino acid, adding at least one amino acid, or the substitution of at least one amino acid. A nucleotide can be added at the C-terminus, the N-terminus, or at internal points within the gene, e.g., to achieve the change in the protein. In a preferred embodiment, the protein naturally expressed in the cell is changed by adding at least one amino acid to the protein, e.g., an added amino acid or amino acids that can bind to a preselected ligand, e.g., a 6X HIS ligand, a cellulose binding domain (CBD) ligand, a maltose binding protein (MBP) ligand. In a preferred embodiment, the protein naturally expressed in the cell is changed by addition of an affinity tag to the gene encoding the naturally expressed protein. In a preferred embodiment, more than 1-100, 5-50, 10-30 amino acid residues can be added to the protein. The residues allow for a physical difference between the altered protein and the heterologous product and are useful, e.g., in purification.

In a preferred embodiment, the protein naturally expressed in the cell is altered by changing the gene encoding the protein such that the stop codon of the protein is altered, e.g., the stop codon is altered by substitution of at least one nucleotide in the stop codon. In another preferred embodiment, the stop codon is altered by removal of at least one nucleotide or addition of at least one nucleotide in the sequence encoding the stop codon.

In a preferred embodiment, the protein naturally expressed in the cell is altered by changing an amino acid sequence of the protein such that the stop codon is altered, e.g., the stop codon is altered by addition of at least one amino acid. In a preferred embodiment, the amino acid is added using a suppressor tRNA and a transferase enzyme. In a preferred embodiment, the cell includes one or more of: a sequence encoding a suppressor tRNA; a sequence encoding a transferase enzyme; a sequence encoding a suppressor tRNA and a sequence encoding a transferase enzyme; a sequence encoding a suppresor tRNA under the control of a promoter; a transgenic sequence encoding a transferase enzyme under the control of a promoter.

In a preferred embodiment, the cell is: a eukaryotic cell. In a preferred embodiment, the cell is of fungal, plant or animal origin, e.g., vertebrate origin. In a preferred embodiment, the cell is: a mammalian cell, e.g., a primary or secondary mammalian cell, e.g., a fibroblast, a hematopoietic stem cell, a myoblast, a keratinocyte, an epithelial cell, an endothelial cell, a glial cell, a neural cell, a cell comprising a formed element of the blood, a muscle cell and precursors of these somatic cells; a transformed or immortalized cell line. Preferably, the cell is a human cell. In another embodiment, the cell line can be cell line other than a human cell line, e.g., a CHO cell line, a COS cell line.

In a preferred embodiment, the heterologous polypeptide is a human polypeptide. In a preferred embodiment, the heterologous polypeptide is: a serum protein, a milk protein, a glycosylated or a nonglycosylated protein. A heterologous polypeptide includes, but is not limited to, any of: alpha proteinase inhibitor, alkaline phosphotase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and alpha-antitiypsin.

Also described are non-human animals, sperm, oocytes, embryos, or fetuses which include an altered version of a naturally expressed protein as described herein. The invention further includes cell lines and DNA constructs as described herein.

Also described is a method of evaluating an affinity tag for its utility in separating a heterologous polypeptide and the protein naturally expressed in the non-human transgenic animal. The evaluation can be performed *in vivo* or *in vitro. In vitro* methods can include engineering a gene comprising an affinity tag by standard recombinant methods, obtaining a recombinantly tagged protein and adding both the tagged protein and a heterologous polypeptide into a sample, e.g., a bodily fluid (e.g., milk, blood, urine), and evaluating the level of tagged protein that can be separated from the sample, to thereby evaluate the utility of the tag.

The terms "protein", "polypeptide" and "peptide" are used interchangeably herein.

As used herein, the term "transgenic sequence" refers to a nucleic acid sequence (e.g., encoding one or more human proteins), which is inserted by artifice into a cell. The transgenic sequence, also referred to herein as a transgene, becomes part of the genome of an animal which develops in whole or in part from that cell. In embodiments of the invention, the transgenic sequence is integrated into the chromosomal genome. If the transgenic sequence is integrated into the genome it results, merely by virtue of its insertion, in a change in the nucleic acid sequence of the genome into which it is inserted. A transgenic sequence can be partly or entirely species-heterologous, i.e., the transgenic sequence, or a portion thereof, can be from a species which is different from the cell into which it is introduced. A transgenic sequence can be partly or entirely species-homologous, i.e., the transgenic sequence, or a portion thereof, can be from the same species as is the cell into which it is introduced. If a transgenic sequence is homologous (in the sequence sense or in the species-homologous sense) to an endogenous gene of the cell into which it is introduced, then the transgenic sequence, preferably, has one or more of the following characteristics: it is designed for insertion, or is inserted, into the cell's genome in such a way as to alter the sequence of the genome of the cell into which it is inserted (e.g., it is inserted at a location which differs from that of the endogenous gene or its insertion results in a change in the sequence of the endogenous gene); it includes a mutation, e.g., a mutation which results in misexpression of the transgenic sequence; by virtue of its insertion, it can result in misexpression of the gene into which it is inserted, e.g., the insertion can result in a knockout of the gene into which it is inserted. A transgenic sequence can include one or more transcriptional regulatory sequences and any other nucleic acid sequences, such as introns, that may be necessary for a desired level or pattern of expression of a selected nucleic acid, all operably linked to the selected nucleic acid. The transgenic sequence can include an enhancer sequence and or sequences which allow for secretion.

As used herein, a "transgenic animal" is a non-human animal, e.g., a non-human mammal, e.g., a bovine, a goat, a horse, a sheep, a camel, a llama, a mouse, or a rat, in which one or more, and preferably essentially all, of the cells of the animal include a transgene. The transgene can be introduced into the cell, directly or indirectly by introduction into a precursor of the cell, e.g., by microinjection, transfection or infection, e.g., by infection with a recombinant virus, or by nuclear transfer. The term genetic manipulation is directed to the introduction of a recombinant DNA molecule. This molecule may be integrated within a chromosome, or it may be extrachromosomally replicating DNA. Transgenic animals can be, e.g., heterozygous or homozygous for a transgene.

Other features and advantages of the invention will be apparent from the following description and from the claims.

### Detailed Description of the Invention

### Methods of Altering a Protein Naturally Expressed in an Animal

Transgenic non-human animals can be produced which include an altered form of a protein naturally expressed by the transgenic animal. Such animals can be used to produce a heterologous polypeptide which can be separated from the host animal's endogenous version of the protein.

The term "altered protein" or "altering a naturally expressed protein" refers to a modification in the gene encoding the protein and/or the amino acid sequence of the protein. Alterations can include, for example, alterations in the gene sequence, or alterations in transcription or translation. As used herein, the term "naturally expressed protein" refers to an endogenous protein of the host animal. For example, bovine serum albumin is an endogenous protein expressed in bovines.

Altering of the host endogenous gene can be done using molecular biological techniques to first clone the genes and then construct DNA molecules that can be introduced into the host animal's cells.

This can be done by replacing the endogenous host gene with a sequence encoding the heterologous polypeptide. The term "replacing" as used herein, refers to "replacement of expression" and is not linked to an "exchange" of one gene for another in the genome. Thus, a DNA sequence, e.g., a cDNA, encoding a heterologous polypeptide can be inserted prior to the initiation codon of the gene encoding the naturally expressed protein with or without removing the sequence coding for the naturally expressed protein from the genome. For example, a cDNA encoding human serum albumin can be inserted into exon 2 of the bovine serum albumin gene without removing the sequence coding for bovine serum albumin for expression in a transgenic bovine. In another preferred embodiment, the gene encoding the naturally expressed protein is exchanged with a DNA sequence, e.g., the genomic sequence, encoding a heterologous polypeptide, e.g., the sequence encoding the naturally expresses protein is removed and replaced with, e.g., the genomic sequence of a heterologous polypeptide. The sequence encoding a heterologous polypeptide can be inserted: after the 5' untranslated region (UTR) of the gene encoding the naturally expressed protein; prior to the 3' UTR of the gene encoding the naturally expressed protein; and/or in between the 5' UTR and the 3' UTR of the gene encoding the naturally expressed protein.

In addition, when the sequence encoding the naturally expressed protein is not removed from the host genome, other modifications can be made to the host's genome. For example, if the host endogenous protein is secreted, the signal sequence can be removed from the protein. In addition, a termination sequence, e.g., a bovine polyA termination sequence, can be inserted onto the '3 end of the cDNA encoding heterologous polypeptide.

Another approach to altering a naturally expressed protein is to place a tag on the endogenous protein which will allow it to be more easily purified away from the transgenic product, i.e., the heterologous polypeptide. The endogenous protein can be altered by changing the sequence of the gene, e.g., by adding, deleting, or substituting a nucleotide, such that the protein is changed. The naturally expressed protein can be changed by, for example, deleting at least one amino acid, adding at least one amino acid, or the substitution of at least one amino acid. A nucleotide can be added at the C-terminus, the N-terminus, or at internal points within the gene, e.g. to achieve the change in the naturally expressed protein.

One approach to changing the endogenous protein is to add at least one amino acid to the protein, e.g., an added amino acid or amino acids that can bind to a preselected ligand. For example, the endogenous protein can be changed by addition of an affinity tag to the gene encoding the endogenous protein. Preferably more than 1-100, 5-50, 10-30 amino acid residues can be added. The residues allow for a physical difference between the altered protein and the heterologous polypeptide. Examples of affinity tags include a 6X HIS tag, a cellulose binding domain (CBD) tag, a maltose binding protein (MBP) and any other peptide that would have a minimal effect on the host animal. An altered protein which includes additional amino acids which can bind a preselected ligand can be used to separate the altered protein from the sample. For example, an altered protein can be separated from a sample by binding the altered protein to a preselected ligand, e.g., a 6X HIS ligand (e.g., a metal chelating column), CBD ligand (e.g., cellulose) or a MBP ligand (e.g., maltose).

Another approach to changing the endogenous protein is to modify the gene encoding the protein such that the stop codon of the bovine serum albumin is altered, e.g., the stop codon is altered by substitution of at least one nucleotide to the stop codon. In another preferred embodiment, the stop codon is altered by removal of at least one nucleotide or addition of at least one nucleotide encoding the stop codon, e.g., removal of at least one nucleotide in the TAA stop codon of the sequence encoding bovine serum albumin. The stop codon can also be altered, at the protein level, by changing an amino acid sequence of the protein, e.g., the stop codon is altered by addition of at least one amino acid. For example, an amino acid can be added using a suppressor tRNA and a transferase enzyme. A transgenic sequence encoding a suppressor tRNA can be expressed in the transgenic mammal. In addition, a transgenic sequence encoding a transferase enzyme can be expressed in a transgenic mammal. Preferably, a transgenic sequence encoding either a suppressor tRNA or a transferase enzyme is under the control of a tissue-specific promoter. Thus, in order to limit the effect of suppressing all proteins which have the particular base codons when a suppressor tRNA is used, a tissue-specific promoter can be used in order to limit expression of the tRNA and transferase enzyme mostly to the desired organ. For example, when the endogenous protein is bovine serum albumin which is produced in the liver, a liver-specific promoter can be used to limit expression of the tRNA to the liver.

Preferably, the suppressor tRNA can recognize at least 2, 3, 4, 5, 6 base codons, e.g., the suppressor tRNA recognizes the TAAAC sequence at the end of the bovine serum albumin coding region.

The methods of altering the naturally expressed protein can be done by manipulating one copy of the endogenous gene, followed by nuclear transfer to obtain an embryo, fetus or adult non-human animal, which can be used to generate a cell line, e.g., a fetal fibroblast cell line. Such cells can be used to produce a transgenic animal that is heterologous for the gene which encodes the altered polypeptide. These cell lines can also be used to manipulate the other wild type copy of the endogenous gene. The resulting cell line should then cany altered endogenous gene in both alleles. Such cells can be used to produced a transgenic non-human animal that is homozygous for the gene which encodes the altered protein.

Either of these cell lines can then be used in the nuclear transfer process to generate the production herd of animals, e.g., cattle or goats, that not only produce the heterologous polypeptide encoded by the transgene, e.g., in their milk, but also carry the altered naturally expressed protein, e.g., in their circulation.

### Transfected Cell Lines

Genetically engineered cells for production of a transgenic non-human mammal can be obtained from a cell line into which a nucleic acid of interest, e.g., a nucleic acid which encodes a protein, has been introduced.

A construct can be introduced into a cell via conventional transformation or transfection techniques. As used herein, the terms "transfection" and "transformation" include a variety of techniques for introducing a transgenic sequence into a host cell, including calcium phosphate or calcium chloride coprecipitation, DEAE-dextrane-mediated transfection, lipofection, or electroporation. In addition, biological vectors, e.g., viral vectors can be used as described below. Suitable methods for transforming or transfecting host cells can be found in Sambrook et al., Molecular Cloning: A Laboratory Manuel, 2nd ed, Cold Spring Harbor Laboratory. (Cold Spring Harbor laboratory Press, Cold Spring Harbor, NY, 1989), and other suitable laboratory manuals.

Two useful approaches are electroporation and lipofection. Brief examples of each are described below.

The DNA construct can be stably introduced into a donor cell line, e.g., an embryonic cell, e.g., an embryonic somatic cell line, by electroporation using the following protocol: the cells are resuspended in PBS at about 4 x 10⁶ cells/ml Fifty micrograms of linearized DNA is added to the 0.5 ml cell suspension, and the suspension is placed in a 0.4 cm electrode gap cuvette (Biorad). Electroporation is performed using a Biorad Gene Pulser electroporator with a 330 volt pulse at 25 mA, 1000 microFarad and infinite resistance. If the DNA construct contains a Neomyocin resistance gene for selection, neomyocin resistant clones are selected following incubation with 350 microgram/ml of G418 (GibcoBRL) for 15 days.

The DNA construct can be stably introduced into a donor cell line by lipofection using a protocol such as the following: about 2 x 10⁵ cells are plated into a 3.5 cmiameter well and transfected with 2 micrograms of linearized DNA using LipfectAMINE^{™} (GibcoBRL). Forty-eight hours after transfection, the cells are split 1:1000 and 1:5000 and, if the DNA construct contains a neomyosin resistance gene for selection, G418 is added to a final concentration of 0.35 mg/ml. Neomyocin resistant clones are isolated and expanded for cryopreservation as well as nuclear transfer.

### Transgenic Mammals

Methods for generating non-human transgenic mammals are known in the art. Such methods can involve introducing DNA constructs into the germ line of a mammal to make a transgenic mammal. For example, one or several copies of the construct may be incorporated into the genome of a mammalian embryo by standard transgenic techniques.

Although bovines and goats are a preferred source of cells, other non-human mammals can be used. Preferred non-human mammals are ruminants, e.g., cows, sheep, camels or goats. Additional examples of preferred non-human animals include oxen, horses, llamas, pigs, mice and rats. The mammal used as the source of cells, e.g., genetically engineered cell, will depend on the transgenic mammal to be obtained. By way of an example, the genome from a bovine should be used from nuclear transfer with a bovine oocyte.

Methods for the preparation of a variety of transgenic animals are known in the art. Protocols for producing transgenic goats are known in the art. For example, a transgene can be introduced into the germline of a goat by microinjection as described, for example, in Ebert et al. (1994) Bio/Technology 12:699. A protocol for the production of a transgenic pig can be found in White and Yannoutsos, Current Topics in Complement Research: 64th Forum in Immunology, pp. 88-94; US Patent No. 5,523,226; US Patent No. 5,573,933; PCT Application WO93/25071; and PCT Application WO95/04744. A protocol for the production of a transgenic rat can be found in Bader and Ganten, Clinical and Experimental Pharmacology and Physiology, Supp. 3:S81-S87, 1996. A protocol for the production of a transgenic cow can be found in U.S. Patent No: 5,741,957, and Transgenic Animal Technology, A Handbook, 1994, ed., Carl A. Pinkert, Academic Press, Inc. A protocol for the production of a transgenic sheep can be found in PCT Publication WO 97/07669, and Transgenic Animal Technology, A Handbook, 1994, ed., Carl A. Pinkert, Academic Press, Inc.

### Tissue-Specific Expression of Proteins

It is often desirable to express a protein, e.g., a heterologous protein, in a specific tissue or fluid, e.g., the milk, blood or urine, of a transgenic non-human animal. The heterologous protein can be recovered from the tissue or fluid in which it is expressed. For example, it is often desirable to express the heterologous protein in milk. Methods for producing a heterologous protein under the control of a milk specific promoter are described below. In addition, other tissue-specific promoters, as well as, other regulatory elements, e.g., signal sequences and sequence which enhance secretion of non-secreted proteins, are described below.

### Milk Specific Promoters

Useful transcriptional promoters are those promoters that are preferentially activated in mammary epithelial cells, including promoters that control the genes encoding milk proteins such as caseins, beta lactoglobulin (Clark et al. (1989) Bio/Technology 7: 487-492), whey acid protein (Gordon et al. (1987) Bio/Technology 5: 1183-1187), and lactalbumin (Soulier et al., (1992) FEBS Letts. 297: 13). Casein promoters may be derived from the alpha, beta, gamma or kappa casein genes of any mammalian species; a preferred promoter is derived from the goat beta casein gene (DiTullio, (1992) Bio/Technology 10:74-77). Milk-specific protein promoter or the promoters that are specifically activated in mammary tissue can be derived from cDNA or genomic sequences. Preferably, they are genomic in origin.

DNA sequence information is available for the mammary gland specific genes listed above, in at least one, and often in several organisms. See, e.g., Richards et al. (1981) J. Biol. Chem. 256, 526-532 (α-lactalbumin rat); Campbell et al. (1984) Nucleic Acids Res. 12, 8685-8697 (rat WAP); Jones et al. (1985) J. Biol. Chem. 260, 7042-7050 (rat β-casein); Yu-Lee & Rosen (1983) J. Biol. Chem. 258, 10794-10804 (rat γ-casein); Hall, Biochem. J. 242, 735-742 (1987) (α-lactalbumin human); Stewart, Nucleic Acids Res. 12, 389 (1984) (bovine αs1 and κ casein cDNAs); Gorodetsky et al.(1988) Gene 66, 87-96 (bovine β casein); Alexander et al. (1988) Eur. J. Biochem. 178, 395-401 (bovine κ casein); Brignon et al. (1977) FEBS Lett. 188, 48-55 (bovine αS2 casein); Jamieson et al. (1987) Gene 61, 85-90, Ivanov et al.(1988) Biol. Chem. Hoppe-Seyler 369, 425-429 (1988), Alexander et al. (1989) Nucleic Acids Res. 17, 6739 (bovine β lactoglobulin); Vilotte et al. (1987) Biochimie 69, 609-620 (bovine α-lactalbumin). The structure and function of the various milk protein genes are reviewed by Mercier & Vilotte (1993) J. Dairy Sci. 76, 3079-3098 (incorporated by reference in its entirety for all purposes). If additional flanking sequences are useful in optimizing expression of the heterologous protein, such sequences can be cloned using the existing sequences as probes. Mammary-gland specific regulatory sequences from different organisms can be obtained by screening libraries from such organisms using known cognate nucleotide sequences, or antibodies to cognate proteins as probes.

### Signal Sequences

Useful signal sequences are milk-specific signal sequences or other signal sequences which result in the secretion of eukaryotic or prokaryotic proteins. Preferably, the signal sequence is selected from milk-specific signal sequences, i.e., it is from a gene which encodes a product secreted into milk. Most preferably, the milk-specific signal sequence is related to the milk-specific promoter used in the construct, which are described below. The size of the signal sequence is not critical. All that is required is that the sequence be of a sufficient size to effect secretion of the desired recombinant protein, e.g., in the mammary tissue. For example, signal sequences from genes coding for caseins, e.g., alpha, beta, gamma or kappa caseins, beta lactoglobulin, whey acid protein, and lactalbumin can be used. A preferred signal sequence is the goat β-casein signal sequence.

Signal sequences from other secreted proteins, e.g., proteins secreted by kidney cells, pancreatic cells or liver cells, can also be used. Preferably, the signal sequence results in the secretion of proteins into, for example, urine or blood.

### Amino-Terminal Regions of Secreted Proteins

A non-secreted protein can also be modified in such a manner that it is secreted such as by inclusion in the protein to be secreted of all or part of the coding sequence of a protein which is normally secreted. Preferably the entire sequence of the protein which is normally secreted is not included in the sequence of the protein but rather only a sufficient portion of the amino terminal end of the protein which is normally secreted to result in secretion of the protein. For example, a protein which is not normally secreted is fused (usually at its amino terminal end) to an amino terminal portion of a protein which is normally secreted.

In one aspect, the protein which is normally secreted is a protein which is normally secreted in milk. Such proteins include proteins secreted by mammary epithelial cells, milk proteins such as caseins, beta lactoglobulin, whey acid protein, and lactalbumin. Casein proteins include alpha, beta, gamma or kappa casein genes of any mammalian species. A preferred protein is beta casein, e.g., goat beta casein. The sequences which encode the secreted protein can be derived from either cDNA or genomic sequences. Preferably, they are genomic in origin, and include one or more introns.

### Other Tissue-Specific Promoters

Other tissue-specific promoters which provide expression in a particular tissue can be used. Tissue specific promoters are promoters which are expressed more strongly in a particular tissue than in others. Tissue specific promoters are often expressed essentially exclusively in the specific tissue. For example, if the altered protein is normally expressed in the liver, a liver-specific promoter can be used. For example, a liver-specific promoter can be used when a suppressor tRNA is used to alter serum albumin. In this situation, a transgenic sequence encoding the suppressor tRNA can be under the control of a liver-specific promoter.

Tissue-specific promoters which can be used include: a neural-specific promoter, e.g., nestin, Wnt-1, Pax-1, Engrailed-1, Engrailed-2, Sonic hedgehog; a liver-specific promoter, e.g., albumin, alpha-1 antitrypsin; a muscle-specific promoter, e.g., myogenin, actin, MyoD, myosin; an oocyte specific promoter, e.g., ZP1, ZP2, ZP3; a testes-specific promoter, e.g., protamin, fertilin, synaptonemal complex protein-1; a blood-specific promoter, e.g., globulin, GATA-1, porphobilinogen deaminase; a lung-specific promoter, e.g., surfactant protein C; a skin- or wool-specific promoter, e.g., keratin, elastin; endothelium-specific promoters, e.g., Tie-1, Tie-2; and a bone-specific promoter, e.g., BMP.

In addition, general promoters can be used for expression in several tissues. Examples of general promoters include β-actin, ROSA-21, PGK, FOS, c-myc, Jun-A, and Jun-B.

### Insulator Sequences

The DNA constructs used to make a transgenic animal can include at least one insulator sequence. The terms "insulator", "insulator sequence" and "insulator element" are used interchangeably herein. An insulator element is a control element which insulates the transcription of genes placed within its range of action but which does not perturb gene expression, either negatively or positively. Preferably, an insulator sequence is inserted on either side of the DNA sequence to be transcribed. For example, the insulator can be positioned about 200 bp to about 1 kb, 5' from the promoter, and at least about 1 kb to 5 kb from the promoter, at the 3' end of the gene of interest. The distance of the insulator sequence from the promoter and the 3' end of the gene of interest can be determined by those skilled in the art, depending on the relative sizes of the gene of interest, the promoter and the enhancer used in the construct. In addition, more than one insulator sequence can be positioned 5' from the promoter or at the 3' end of the transgene. For example, two or more insulator sequences can be positioned 5' from the promoter. The insulator or insulators at the 3' end of the transgene can be positioned at the 3' end of the gene of interest, or at the 3'end of a 3' regulatory sequence, e.g., a 3' untranslated region (UTR) or a 3' flanking sequence.

A preferred insulator is a DNA segment which encompasses the 5' end of the chicken β-globin locus and corresponds to the chicken 5' constitutive hypersensitive site as described in PCT Publication 94/23046, the contents of which is incorporated herein by reference.

### DNA Constructs

A cassette which encodes a heterologous protein can be assembled as a construct which includes a promoter, e.g., a promoter for a specific tissue, e.g., for mammary epithelial cells, e.g., a casein promoter, e.g., a goat beta casein promoter, a milk-specific signal sequence, e.g., a casein signal sequence, e.g., a β-casein signal sequence, and a DNA encoding the heterologous protein.

The construct can also include a 3' untranslated region downstream of the DNA sequence coding for the non-secreted protein. Such regions can stabilize the RNA transcript of the expression system and thus increases the yield of desired protein from the expression system. Among the 3' untranslated regions useful in the constructs for use in the invention are sequences that provide a poly A signal. Such sequences may be derived, e.g., from the SV40 small t antigen, the casein 3' untranslated region or other 3' untranslated sequences well known in the art. In one aspect, the 3' untranslated region is derived from a milk specific protein. The length of the 3' untranslated region is not critical but the stabilizing effect of its poly A transcript appears important in stabilizing the RNA of the expression sequence.

Optionally, the construct can include a 5' untranslated region between the promoter and the DNA sequence encoding the signal sequence. Such untranslated regions can be from the same control region from which promoter is taken or can be from a different gene, e.g., they may be derived from other synthetic, semisynthetic or natural sources. Again their specific length is not critical, however, they appear to be useful in improving the level of expression.

The construct can also include about 10%, 20%, 30%, or more of the N-terminal coding region of a gene preferentially expressed in mammary epithelial cells. For example, the N-terminal coding region can correspond to the promoter used, e.g., a goat β-casein N-terminal coding region.

The construct can be prepared using methods known in the art. The construct can be prepared as part of a larger plasmid. Such preparation allows the cloning and selection of the correct constructions in an efficient manner. The construct can be located between convenient restriction sites on the plasmid so that they can be easily isolated from the remaining plasmid sequences for incorporation into the desired mammal.

### Heterologous Proteins

Transgenic sequences encoding heterologous proteins can be introduced into the germline of a non-human mammal or can be transfected into a cell line as described above. The protein can be a complex or multimeric protein, e.g., a homo- or heteromultimer, e.g., proteins which naturally occur as homo- or heteromultimers, e.g., homo- or hetero- dimers, trimers or tetramers. The protein can be a protein which is processed by removal, e.g., cleavage, of N-terminus, C-terminus or internal fragments. Even complex proteins can be expressed in active form. Protein encoding sequences which can be introduced into the genome of mammal, e.g., bovines or goats, include a serum protein, a milk protein, a glycosylated or a non-glycosylated protein. The protein may be human or non-human in origin. The heterologous protein may be a potential therapeutic or pharmaceutical agent such as, but not limited to: alpha-1 proteinase inhibitor, alpha-1 antitrypsine, alkaline phosphatase, angiogenin, antithrombin III, any of the blood clotting factors including Factor VIII, Factor IX, and Factor X chitinase, erythropoietin, extracellular superoxide dismutase, fibrinogen, glucocerebrosidase, glutamate decarboxylase, human growth factor, human serum albumin, immunoglobulin, insulin, myelin basic protein, proinsulin, prolactin, soluble CD4 or a component or complex thereof, lactoferrin, lactoglobulin, lysozyme, lactalbumin, tissue plasminogen activator or a variant thereof.

Nucleotide sequence information is available for several of the genes encoding the heterologous proteins listed above, in at least one, and often in several organisms. *See e.g*., Long et al. (1984) Biochem. 23(21):4828-4837 (aplha-1 antitrypsin); Mitchell et al. (1986) Prot. Natl. Acad. Sci USA 83:7182-7186 (alkaline phosphatase); Schneider et al. (1988) EMBO J. 7(13):4151-4156 (angiogenin); Bock et al. (1988) Biochem. 27(16):6171-6178 (antithrombin III); Olds et al. (1991) Br. J. Haematol. 78(3):408-413 (antithrombin III); Lin et al. (1985) Proc. Natl. Acad. Sci. USA 82(22):7580-7584 (erythropoeitin); U.S. Patent No. 5,614,184 (erythropoietin); Horowitz et al. (1989) Genomics 4(1):87-96 (glucocerebrosidase); Kelly et al. (1992) Ann. Hum. Genet. 56(3):255-265 (glutamte decarboxylase); U.S. Patent No. 5,707,828 (human serum albumin); U.S. Patent No. 5,652,352 (human serum albumin); Lawn et al. (1981) Nucleic Acid Res. 9(22):6103-6114 (human serum albumin); Kamholz et al. (1986) Prot. Natl. Acad. Sci. USA 83(13):4962-4966 (myelin basic protein); Hiraoka et al. (1991) Mol. Cell Endocrinol. 75(1):71-80 (prolactin); U.S. Patent No. 5,571,896 (lactoferrin); Pennica et al. (1983) Nature 301(5897):214-221 (tissue plasminogen activator); Sarafanov et al. (1995) Mol. Biol. 29:161-165, the contents of which are incorporated herein by reference.

### Examples

### Replacement of bovine serum albumin (BSA) with human serum albumin (HSA)

One method of reducing the contaminating BSA in milk is to replace it with the human version, i.e., HSA. Since the human and bovine serum albumin proteins are very similar and since they do not carry out enzymatic functions, human serum albumin can replace BSA in the animal.

Serum albumin is normally produced in the liver and is found at high levels in the serum. In this series of experiments, the bovine gene that codes for BSA is replaced with the gene encoding the human version. The resulting animals produce high levels of HSA in their bloodstream. Any serum albumin that then leaks into the mammary gland is HSA. Since serum albumin has no glycosylation sites, the HSA produced in the liver and found in the serum is identical to that produced in the mammary gland by the milk-specific promoters. This process requires the cloning the genes for human and bovine serum albumin. The human gene is then introduced into the bovine genome in a manner to replace the cow's own gene. This process takes place in cells grown in culture that is eventually be used to generate live cattle by a process of nuclear transfer.

### Cloning of the cDNA sequence for BSA

The sequence of BSA mRNA is published in GenBank as M73993 which contains 2035 bp of sequence. The mRNA is likely to be identical in different lines of cattle. The cDNA from a commercial bovine library can be cloned and sequenced to confirm that it is correct. The cDNA can then be used in generating both BSA Tag versions and in the replacement process.

### Cloning of BSA genomic sequence

The cDNA sequence can be used as a probe to clone the genomic version of BSA. Although the sequence coding for the BSA protein should be identical, the level of heterogeneity of the intron and flanking regions can be determined and used as a target sequence for homologous recombination. Preferably, the cell line is one that has already given rise to an animal that produces HSA in its milk. The gene from founder animals which were derived by microinjection of a transgene and which carry the construct enabling them to produce HSA in their milk can be used.

### Construction of HSA cDNA in Exon I of BSA

One means of replacing the BSA is to place the cDNA sequence encoding HSA just 5' of the initiating codon of BSA which is found in Exon 2 of the gene. By using oligomutagenesis, an *XhoI* site can be introduced into Exon 2. In addition, the signal sequence of BSA can be removed. For example, a construct which includes a cDNA version of HSA that carries an optimized Kozak sequence and is contained in a 1.5 kb Xho1 fragment can be used. Earlier work has shown that this cDNA sequence can be expressed at high levels in transgenic animals. Transcription occurs through this hybrid gene and the HSA is secreted, but the BSA coding sequence, which has been disrupted, is not.

In order to more fully insure no expression of the BSA gene, the bovine bgh polyA termination sequence can be inserted on the 3' end of the inserted HSA cDNA. This should end transcription at the end of the HSA gene. The remaining portion of BSA is not transcribed and, therefore, is silent.

### Construction of HSA replacement of BSA coding sequence

An alternative method of HSA replacement of BSA, is to replace the coding region of the genomic BSA with the genomic version of HSA. This method includes introduction of the same unique cloning sites into the untranslated region (UTR) of both genomic HSA and genomic BSA. The coding sequence of HSA is then cloned into the corresponding regions of BSA. The resulting vectors, therefore, contain the coding region of HSA flanked by the UTR of BSA. These BSA UTRs can be used for homology to carry out recombination in which the genomic coding region of BSA is replaced by HSA. This gene construct can be used for high-level expression by the endogenous bovine promoter.

### Addition of An Affinity Tag to BSA

Since it is often necessary to remove the BSA from the process to such a low level, an affinity tag can be placed on this protein that allows for tight binding in the presence of HSA. An example of such an affinity tag is a 6 X HIS tag, which can be used to bind proteins at nanogram levels. Proteins with this tag can be isolated by their ability to bind to a metal chelating column. Other affinity tags can be utilized as well. Examples of such tags include cellulose binding domain (CBD), maltose binding protein (MBP), or any peptide that would have minimal effect on the host animal.

### BSA 6X HIS Fusion

Since the sequence of BSA is known, it is possible to design oligonucleotides that can be used to add a 6X HIS tag to the C-terminus of the BSA coding region. Once the cDNA of BSA has been cloned, the 6X HIS tag is ligated to the C-terminus using standard techniques.

### Expression of the BSA Tag

The BSA Tag can be expressed both in tissue culture and in the milk of transgenic mice. This allows for the ability of the new version of BSA to bind to metal chelating columns to be tested. It also can be used to show that the BSA 6X HIS Tag is capable of being secreted from mammalian cells.

### Test for HSA protein binding to Chelating Column

Human serum albumin protein can be tested for its binding capacity to the metal chelating columns.

### Comparison of HSA and BSA Tag

The experiment can be used to compare the relative binding of the two proteins in a purification process. The 6X HIS tag allows for removal of the BSA from a solution of HSA to levels required by FDA standards, (1 ppm). Briefly, HSA is added to cow milk at 10 g/L. The BSA Tag is added to the mixture to 0.2 g/L. A mock purification can be carried out, similar to the processes already described for HSA purification. Using such a method, the level the BSA Tag can be tested to determine if it can be removed to less than 1 ppm level.

### Addition of CBD fusion tag

Another tag that can be added to BSA is the affinity protein for cellulose binding, CBD. The 15kd portion of the enzyme for cellulose degradation contains the region of the enzyme that specifically binds to cellulose.

### CBD BSA cDNA Construct

Utilizing the cDNA for BSA, the coding region of the CBD protein fragment can be linked in frame to the BSA gene. A version of the CBD protein has been designed that utilizes the codon preference of the mammary gland. In addition, the glycosylation sites have been removed allowing more effective binding to cellulose. A CBD fusion of this peptide has been successfully expressed in both tissue culture and into the milk of transgenic mice. Using standard techniques, the CBD gene can be linked in frame to the coding region of BSA, such that a fusion protein can be produced.

### Expression of BSA-CBD fusion

This protein can be expressed in both tissue culture and in milk of transgenic mice. It is expected that the protein can specifically bind to cellulose. This can be tested by the use of cellulose beads to specifically bind the fusion protein. It is important that a cellulose process can scrub all of the BSA-CBD out of a milk solution that contains high levels of HSA. It is also necessary to show that the fusion protein is capable of being expressed at high levels. Since this fusion protein is replacing the animal's normal serum albumin, it is necessary to have it expressed as readily as BSA.

### Effectiveness of Binding

The BSA-CBD protein can be produced in both tissue and in milk. The protein can be tested for its ability to bind to cellulose in the presence of high levels of HSA. This can be done on a pilot scale using low levels of BSA-CBD. Assays can be used which detect ppm levels of BSA in the presence of HSA. The cellulose needs to remove the BSA-CBD to these levels.

### Changing the BSA protein (BSA)

Another method of changing the characteristics of BSA to make it more easily separated or purified from HSA is to add a peptide tail. Due to limitations on the size of DNA that can be used in homologous recombination, very small segments of DNA may be needed to effect changes in the BSA molecule. Since the sequence of BSA and its 3'UTR is known, this can be accomplished by eliminating the stop codon TAA. This gives rise to an extended version of BSA that is translated until the next stop codon is reached. Depending upon which reading frame is chosen, the downstream fusion peptide can be of three different types. They range in size from 11, 20 or 45 additional amino acids. These forms of BSA are altered in their physical properties such that they are more easily purified from the HSA.

### Testing the Extended BSA

The cDNA of BSA sequence is published and the clone can be obtained. Using oligo mutagenesis, the TAA stop codon can be eliminated along with the number of bp to put the downstream sequence into each of the three reading frames. These three extended BSA molecules can then be expressed in tissue culture.

### Separation Characteristics

Each version can be tested for its purification characteristics. It is preferable that the new BSA is separated from the HSA during the processing steps, thereby eliminating the need for a step that specifically removes the BSA.

### Effects on the host animal

Another important aspect is whether the BSA is easily secreted. Since this version of BSA is the only one that the animal carries, it is important that it be capable of expression at the same level as normal BSA.

Animals can be produced that will first carry one altered allele. These animals can be monitored to test any effects due to altered BSA. In addition, the gene can be used to generate transgenic mice that over express the protein in their liver. These animals can also be tested for effects due to altered BSA.

### Dominant Change in BSA

Due to the time required to generate homozygous cattle, an alternative can be attempted to alter the BSA by the introduction of additional genes that will function to change the BSA expression. This dominant phenotype can significantly change the timelines, in that only heterozygous animals would be needed to start production. The TAA stop codon can be changed utilizing a Suppressor tRNA.

### Suppressor tRNA

Suppressor tRNA molecules function by introducing an amino acid at a stop codon. They have been expensively studied in bacterial genetics and are being developed to function in mammalian cells. The tRNA also requires its complementary amino acid transferase enzyme that adds the amino acid to the tRNA. Since this protein is not present in mammalian cells, this gene must be introduced for the Suppressor tRNA to function.

### Suppressor tRNA to produce BSA Extended

An extended version of BSA can be produced if the stop codon were removed from the BSA gene. A Suppressor tRNA specific to that TAA stop codon, can accomplish that by introducing an amino acid at that site. If the tRNA Suppressor and its corresponding transferase are expressed at high levels in the animal's liver, the BSA produced may be of the BSA, extended form. This requires dominant expression of these new genes such that all of the BSA mRNA produced in the liver is acted upon by the suppressor TRNA. The suppression of stop codons in the organism may have adverse effects. This can be avoided by limiting expression of the suppressor tRNA only to required organs. Since BSA is produced in the liver, the additional genes can be expressed from a liver specific promoter, e.g., the BSA promoter.

### Modifying the Suppressor tRNA

There are many proteins expressed in the liver, and certainly many may have TAA as their stop codon. In order to limit the effect of suppressing all of these TAA stop codons, the suppressor tRNA can be altered. It is known that tRNAs can be changed so that they recognize 2 or 4 base codons instead of the normal triplet. Since the sequence at the end of the BSA coding region is TAAAC, a tRNA that can recognize the TAAAC sequence can be designed. This provides more specific suppression to the BSA.

### Test Model for tRNA Suppression

It is necessary to test for a suppressor's effectiveness in a model system. To do this the genes can be expressed in the mammary gland of the mouse. These genes can be linked to the milk promoters. Transgenic mice that carry the BSA, the suppressor tRNA, and its transferase enzyme, can be generated. When these three genes are expressed, the effectiveness of suppression of the BSA can be determined. The relative expression of each of the genes, as well as, the proportion of BSA Extended produced can be measured and compared to the normal BSA.

## Claims

1. A method of providing an exogenous polypeptide expressed by a non-human transgenic mammal, wherein the exogenous polypeptide is homologous to an endogenous polypeptide expressed by the mammal, the method comprising:
providing a transgenic mammal which comprises a nucleic acid sequence encoding the exogenous polypeptide of a different species than the transgenic mammal, the transgenic mammal also expressing an endogenous form of the polypeptide, wherein the endogenous polypeptide has been altered from the endogenous form naturally expressed by the transgenic mammal and wherein the altered endogenous polypeptide is not the same as the exogenous polypeptide;
obtaining a sample from the transgenic mammal, wherein the sample comprises both the exogenous polypeptide and the altered endogenous polypeptide; and
separating the altered endogenous polypeptide from the sample,
thereby providing the exogenous polypeptide in the sample.

2. The method of claim 1, wherein the sample is milk.

3. The method of claim 1, wherein the non-human transgenic mammal is homozygous for a nucleic acid which encodes the altered polypeptide.

4. The method of claim 1, wherein the non-human transgenic mammal is heterozygous for a nucleic acid which encodes the altered polypeptide.

5. The method of claim 1, wherein the polypeptide naturally expressed in the non-human transgenic mammal can be altered by deleting at least one amino acid, adding at least one amino acid, or substituting at least one amino acid.

6. The method of claim 5, wherein at least one amino acid is added and the added amino acid or amino acids can bind to a preselected ligand.

7. The method of claim 6, wherein the preselected ligand is selected from the group consisting of: a 6X HIS ligand, a cellulose binding domain (CBD) ligand, and a maltose binding protein (MBP) ligand.

8. The method of claim 5, wherein the polypeptide naturally expressed in the non-human transgenic mammal is altered by addition of an affinity tag to the nucleic acid encoding the naturally expressed polypeptide.

9. The method of claim 1, wherein the polypeptide naturally expressed in the non-human transgenic mammal is altered by changing a nucleic acid encoding the naturally expressed polypeptide such that a stop codon of the polypeptide is altered.

10. The method of claim 9, wherein the stop codon is altered by substitution of at least one nucleotide in the stop codon.

11. The method of claim 9, wherein the stop codon is altered by removal of at least one nucleotide or addition of at least one nucleotide in the sequence encoding the stop codon.

12. The method of claim 1, wherein the polypeptide naturally expressed in the non-human transgenic mammal is altered by changing a nucleic acid sequence coding for the polypeptide such that a stop codon is altered.

13. The method of claim 5, wherein the polypeptide naturally expressed by the non-human transgenic mammal is altered by addition of at least one nucleotide triplet coding for an amino acid prior to a stop codon in the polynucleotide.

14. The method of claim 1, wherein the transgenic mammal is selected from the group consisting of a goat, a bovine, a sheep, a pig, a horse, a mouse, and a rat.

15. The method of claim 1, wherein the transgenic mammal is a cow.

16. The method of claim 1, wherein the transgenic mammal is a goat.

17. The method of claim 1, wherein the exogenous polypeptide is a human polypeptide.

18. The method of claim 1, wherein the exogenous polypeptide is selected from the group consisting of: alpha proteinase inhibitor, alkaline phosphatase, angiogenin, extracellular superoxide dismutase, fibrogen, glucocerebrosidase, glutamate decarboxylase, human serum albumin, myelin basic protein, proinsulin, soluble CD4, lactoferrin, lactoglobulin, lysozyme, lactoalbumin, erythrpoietin, tissue plasminogen activator, human growth factor, antithrombin III, insulin, prolactin, and alpha-antitrypsin.

19. The method of claim 1, wherein the exogenous polypeptide is human serum albumin.

20. The method of claim 1, wherein the exogenous sequence is under the control of a tissue-specific promoter.

21. The method of claim 20, wherein the promoter is a milk specific promoter.

22. The method of claim 1, wherein the milk-specific promoter is selected from the group consisting of: a casein promoter, a beta lactoglobulin promoter, a whey acid protein promoter, and a lactalbumin promoter.

## Patentansprüche

1. Verfahren zur Bereitstellung eines exogenen Polypeptids, das durch ein nichthumanes transgenes Säugetier exprimiert wird, wobei das exogene Polypeptid zu einem durch das Säugetier exprimierten endogenen Polypeptid homolog ist: wobei das Verfahren aufweist:
Bereitstellen eines transgenen Säugetiers, das eine Nucleinsäuresequenz aufweist, die das exogene Polypeptid einer anderen Spezies als der des transgenen Säugetiers codiert, wobei das transgene Säugetier außerdem eine endogene Form des Polypeptids exprimiert, wobei das endogene Polypeptid gegenüber der durch das transgene Säugetier natürlich exprimierten endogenen Form verändert worden ist, und wobei das veränderte endogene Polypeptid nicht identisch mit dem exogenen Polypeptid ist;
Entnahme einer Probe von dem transgenen Säugetier, wobei die Probe sowohl das exogene Polypeptid als auch das veränderte endogene Polypeptid aufweist; und
Trennen des veränderten endogenen Polypeptids von der Probe,
wodurch das exogene Polypeptid in der Probe bereitgestellt wird.

2. Verfahren nach Anspruch 1, wobei die Probe Milch ist.

3. Verfahren nach Anspruch 1, wobei das nichthumane transgene Säugetier homozygot für eine Nucleinsäure ist, die das veränderte Polypeptid codiert.

4. Verfahren nach Anspruch 1, wobei das nichthumane transgene Säugetier heterozygot für eine Nucleinsäure ist, die das veränderte Polypeptid codiert.

5. Verfahren nach Anspruch 1, wobei das in dem nichthumanen transgenen Säugetier natürlich exprimierte Polypeptid durch Deletieren mindestens einer Aminosäure, Hinzufügen mindestens einer Aminosäure oder Substituieren mindestens einer Aminosäure verändert werden kann.

6. Verfahren nach Anspruch 5, wobei mindestens eine Aminosäure hinzugefügt wird und die hinzugefügte(n) Aminosäure(n) sich an einen vorgewählten Liganden binden kann (können).

7. Verfahren nach Anspruch 6, wobei der vorgewählte Ligand aus der Gruppe ausgewählt ist, die aus einem 6X HIS-Liganden, einem Liganden mit Cellulose-Bindedomäne (CBD) und einem Liganden mit Maltose-Bindeprotein (MBP) besteht.

8. Verfahren nach Anspruch 5, wobei das in dem nichthumanen transgenen Säugetier natürlich exprimierte Polypeptid durch Hinzufügen einer Affinitätsmarkierung zu der das natürlich exprimierte Polypeptid codierenden Nucleinsäure verändert wird.

9. Verfahren nach Anspruch 1, wobei das in dem nichthumanen transgenen Säugetier natürlich exprimierte Polypeptid verändert wird, indem eine das natürlich exprimierte Polypeptid codierende Nucleinsäure so verändert wird, dass ein Stoppcodon des Polypeptids verändert wird.

10. Verfahren nach Anspruch 9, wobei das Stoppcodon durch Substitution mindestens eines Nucleotids in dem Stoppcodon verändert wird.

11. Verfahren nach Anspruch 9, wobei das Stoppcodon durch Entfernen mindestens eines Nucleotids oder Hinzufügen mindestens eines Nucleotids in der das Stoppcodon codierenden Sequenz verändert wird.

12. Verfahren nach Anspruch 1, wobei das in dem nichthumanen transgenen Säugetier natürlich exprimierte Polypeptid verändert wird, indem eine das Polypeptid codierende Nucleinsäuresequenz so verändert wird, dass ein Stoppcodon verändert wird.

13. Verfahren nach Anspruch 5, wobei das durch das nichthumane transgene Säugetier natürlich exprimierte Polypeptid verändert wird, indem mindestens ein Nucleotidtriplett, das eine Aminosäure codiert, vor einem Stoppcodon in dem Polynucleotid eingefügt wird.

14. Verfahren nach Anspruch 1, wobei das transgene Säugetier aus der Gruppe ausgewählt ist, die aus einer Ziege, einem Rind, einem Schaf, einem Schwein, einem Pferd, einer Maus und einer Ratte besteht.

15. Verfahren nach Anspruch 1, wobei das transgene Säugetier eine Kuh ist.

16. Verfahren nach Anspruch 1, wobei das transgene Säugetier eine Ziege ist.

17. Verfahren nach Anspruch 1, wobei das exogene Polypeptid ein humanes Polypeptid ist.

18. Verfahren nach Anspruch 1, wobei das exogene Polypeptid aus der Gruppe ausgewählt ist, die aus α-Proteinaseinhibitor, alkalischer Phosphatase, Angiogenin, extrazellulärer Superoxiddismutase, Fibrogen, Glucocerebrosidase, Glutamatdecarboxylase, Humanserumalbumin, basischem Myelinprotein, Proinsulin, löslichem CD4, Lactoferrin, Lactoglobulin, Lysozym, Lactoalbumin, Erythropoietin, Gewebe-Plasminogenaktivator, humanem Wachstumsfaktor, Antithrombin III, Insulin, Prolactin und α-Antitrypsin besteht.

19. Verfahren nach Anspruch 1, wobei das exogene Polypeptid Humanserumalbumin ist.

20. Verfahren nach Anspruch 1, wobei die exogene Sequenz unter der Kontrolle eines gewebespezifischen Promotors steht.

21. Verfahren nach Anspruch 20, wobei der Promotor ein milchspezifischer Promotor ist.

22. Verfahren nach Anspruch 1, wobei der milchspezifische Promotor aus der Gruppe ausgewählt ist, die aus einem Kasein-Promotor, einem β-Lactoglobulin-Promotor, einem Molkensäureprotein-Promotor und einem Lactalbumin-Promotor besteht.

## Revendications

1. Méthode permettant d'obtenir un polypeptide exogène exprimé par un mammifère transgénique non humain, dans laquelle le polypeptide exogène est homologue à un polypeptide endogène exprimé par le mammifère, la méthode comprenant:
l'utilisation d'un mammifère transgénique qui comprend une séquence d'acide nucléique codant pour le polypeptide exogène d'une espèce différente de celle du mammifère transgénique, le mammifère transgénique exprimant également une forme endogène du polypeptide, où le polypeptide endogène a été modifié par rapport à la forme endogène exprimée naturellement par le mammifère transgénique et où le polypeptide endogène modifié n'est pas le même que le polypeptide exogène;
l'obtention d'un échantillon du mammifère transgénique, où l'échantillon comprend à la fois le polypeptide exogène et le polypeptide endogène modifié; et
la séparation du polypeptide endogène modifié contenu dans l'échantillon,
permettant ainsi d'obtenir le polypeptide exogène dans l'échantillon.

2. Méthode de la revendication 1, où l'échantillon est un échantillon de lait.

3. Méthode de la revendication 1, où le mammifère transgénique non humain est homozygote pour un acide nucléique qui code pour le polypeptide modifié.

4. Méthode de la revendication 1, où le mammifère transgénique non humain est hétérozygote pour un acide nucléique qui code pour le polypeptide modifié.

5. Méthode de la revendication 1, où le polypeptide exprimé naturellement chez le mammifère transgénique non humain peut être modifié par la délétion d'au moins un acide aminé, l'addition d'au moins un acide aminé ou la substitution d'au moins un acide aminé.

6. Méthode de la revendication 5, où au moins un acide aminé est ajouté et où l'acide aminé ou les acides aminés ajouté(s) peu(ven)t se lier à un ligand présélectionné.

7. Méthode de la revendication 6, où le ligand présélectionné est sélectionné dans le groupe consistant en: un ligand 6x His, un ligand de type CBD (domaine de liaison à la cellulose) et un ligand de type MBP (protéine liant le maltose).

8. Méthode de la revendication 5, où le polypeptide exprimé naturellement chez le mammifère transgénique non humain est modifié par l'addition d'un marqueur d'affinité sur l'acide nucléique codant pour le polypeptide exprimé naturellement.

9. Méthode de la revendication 1, où le polypeptide exprimé naturellement chez le mammifère transgénique non humain est modifié par un changement dans un acide nucléique codant pour le polypeptide exprimé naturellement tel qu'un codon de terminaison du polypeptide est modifié.

10. Méthode de la revendication 9, où le codon de terminaison est modifié par la substitution d'au moins un nucléotide dans le codon de terminaison.

11. Méthode de la revendication 9, où le codon de terminaison est modifié par l'élimination d'au moins un nucléotide ou l'addition d'au moins un nucléotide dans la séquence codant pour le codon de terminaison.

12. Méthode de la revendication 1, où le polypeptide exprimé naturellement chez le mammifère transgénique non humain est modifié par un changement dans une séquence d'acide nucléique codant pour le polypeptide tel qu'un codon de terminaison est modifié.

13. Méthode de la revendication 5, où le polypeptide exprimé naturellement chez le mammifère transgénique non humain est modifié par l'addition d'au moins un triplet nucléotidique codant pour un acide aminé situé en amont d'un codon de terminaison dans le polynucléotide.

14. Méthode de la revendication 1, où le mammifère transgénique est sélectionné dans le groupe consistant en une chèvre, un bovin, un mouton, un porc, un cheval, une souris et un rat.

15. Méthode de la revendication 1, où le mammifère transgénique est une vache.

16. Méthode de la revendication 1, où le mammifère transgénique est une chèvre.

17. Méthode de la revendication 1, où le polypeptide exogène est un polypeptide humain.

18. Méthode de la revendication 1, où le polypeptide exogène est sélectionné dans le groupe consistant en: un inhibiteur de la protéinase alpha, la phosphatase alcaline, l'angiogénine, la superoxyde dismutase extracellulaire, le fibrogène, la glucocérébrosidase, la glutamate décarboxylase, l'albumine sérique humaine, la protéine basique de la myéline, la proinsuline, les CD4 solubles, la lactoferrine, la lactoglobuline, le lysozyme, la lactoalbumine, l'érythropoïétine, l'activateur tissulaire du plasminogène, le facteur de croissance humain, l'antithrombine III, l'insuline, la prolactine et l'antitrypsine alpha.

19. Méthode de la revendication 1, où le polypeptide exogène est l'albumine sérique humaine.

20. Méthode de la revendication 1, où la séquence exogène est sous le contrôle d'un promoteur spécifique d'un tissu.

21. Méthode de la revendication 20, où le promoteur est un promoteur spécifique du lait.

22. Méthode de la revendication 1, où le promoteur spécifique du lait est sélectionné dans le groupe consistant en: un promoteur de la caséine, un promoteur de la bêta-lactoglobuline, un promoteur de la protéine acide du lactosérum et un promoteur de la lactoalbumine.
